# EUROPEAN PATENT APPLICATION

(11) **EP 4 227 400 A1**
(43) Date of publication of application: **16.08.2023**
(21) Application number: 22156149.1
(22) Date of filing: 10.02.2022
(51) Int. Cl.: C12N 1/14, C12N 13/00, A01G 18/00

(54) **PRODUCTION OF A MYCELIUM-BASED BUILDING ELEMENT**

(71) Applicant: MyCellTech B.V., 7827 TB Emmen (NL)
(72) Inventor: van Driel, Roland, 7827 TB Emmen (NL); Borra, Hans Antonius, 3707 TB Zeist (NL)
(74) Representative: De Vries & Metman

(57) **Abstract**

Process of making a mycelium based material in a mould. Electrical stimulation is used to control growth of the mycelium, in particular to stimulate mycelium growth during colonization of the substrate and/or to inhibit further growth in a deactivating step deactivating the mycelium.

## Description

The present invention relates to a production process for a mycelium-based building element, such as a building panel or insulation panel, e.g., for insulating walls or roofs.

WO 2008/073489 discloses a method of making a composite material comprising the steps of:
- forming an inoculum including a preselected fungus;
- forming a mixture of a substrate of discrete particles and a nutrient material, said nutrient material being capable of being digested by said fungi;
- adding the inoculum to the mixture; and
- allowing said fungus to digest the nutrient over a period sufficient to grow hyphae and to allow the hyphae to form a network of interconnected mycelia cells through and around the discrete particles thereby bonding said discrete particles together to form a self-supporting composite material. Formation of fruiting bodies (mushrooms) must be avoided, so as to obtain a substantially homogeneous composite. The mycelium is subsequently deactivated, e.g., by dehydration to inhibit further growth. The composite can for example be used as a building element, such as a panel. Due to the relatively long steps of mycelial colonization and deactivation, this production process is rather slow and therefore economically less attractive.

It is an object of the present invention to provide a process for the production of a mycelium-based building element with shorter production cycles.

The object of the invention is achieved with a process of making a mycelium based material comprising the steps of:
- depositing a substrate particulate material in a mould to form a substrate bed, the substrate particulate material comprising nutrients capable of being digested by a selected mycelium;
- inoculating a substrate material with a mycelial inoculum to initiate colonization of the substrate bed by the mycelium;
- in a colonization step allowing the mycelium to colonize the inoculated substrate bed to form a mycelial network bonding the substrate particulate material;
- in a deactivating step deactivating the mycelium to inhibit further growth;
wherein electrical currents are used to control growth of the mycelium.

It was found that the time needed for full mycelial colonization can be reduced by as much as about 50 % and production cycles can significantly be shortened.

The article *"*Effect of Electrical Stimulation on Fruit Body Formation in Cultivating Mushrooms" by Takaki c.s., Microorganisms, 2014, discloses a method of stimulating fruit body formation in cultivating mushrooms. Similarly, the article *"*Electrical stimulation for the growth of plants: With special attention to the effects of nearby lightning on mushrooms" by Jamil c.s., in Asian Journal of Microbiology, Biotechnology and Environmental Sciences, 2018, disclose the use of electric stimulation to improve mushroom production. These publications do not relate to the production of mycelium composites or building elements, and teach away from deactivating the mycelium after completing colonization.

During the colonization step conditions are controlled to promote mycelium growth within the substrate without producing mushrooms, which are the fruiting bodies of the mycelium. To this end the conditions are controlled to simulate summer conditions, rather than autumn conditions which promote the production of fruiting bodies. For instance, the CO2 concentration in the cultivation room can be kept above 2000 ppm to stimulate growth. An optimum speed in growth ecan for example be achieved when the Co2 concentration is within the range 10.000 to 20.000 ppm. The relative humidity can for example be kept at 60% to 70%. The O2 concentration can for example be kept between 2.000 ppm and 20.000 ppm. The temperature can for example be kept between 20 and 25 °C.

It was found that duration of the colonization step can be reduced with about 50 % if electrical currents are generated across the substrate bed during the colonization step, the currents having an amperage below 20 A, e.g., below 10 A. A voltage can be applied across the substrate bed, for example below 1000 Volt, e.g., below 100 Volt.

It was found that electrical currents promote mycelial growth, but surprisingly they also inhibit further mycelial growth after colonization of the substrate, in particular if currents are generated across the mycelium composite during the deactivating step having an amperage of at least 20 A, e.g., at least 30 A. The voltage applied during the deactivating step can for example be at least 10 Volt, e.g., at least 100 Volt.

In the colonization step and/or the deactivating step, the currents can for example be generated for at least 5 seconds, e.g., at least 10 seconds. The currents can be generated in pulses or continuously.

The currents can be generated by electrodes at a bottom of the mould and on top of the substrate particulate material. The electrodes can for example be plate electrodes or grid electrodes covering the top and bottom surfaces of the substrate particulate material. Alternatively, the electrodes can be pin electrodes distributed over the top and bottom sides of the substrate bed and protruding into the substrate material.

Good results are obtained with mycelium selected from the following strains: Ganoderma Lucidem; Ganoderma lipsiense; Ganoderma adspersum; Pleurotus ostreatus; Pleurotus eryngii; Schizophyllum commune; Picnoporus sanguineus; Stropharia rugosoannulata. Other strains can also be used.

The above-described aspects are further explained with reference to the drawings.
Figure 1A: Shows an exemplary set-up for carrying out a process of the invention;
Figure 1B: shows the set-up of Figure 1A in exploded view;
Figure 2A: Shows a second exemplary set-up for carrying out a process of the invention;
Figure 2B: shows the set-up of Figure 2A in exploded view;
Figure 3A: Shows a third exemplary set-up for carrying out a process of the invention;
Figure 3B: shows the set-up of Figure 3A in exploded view.

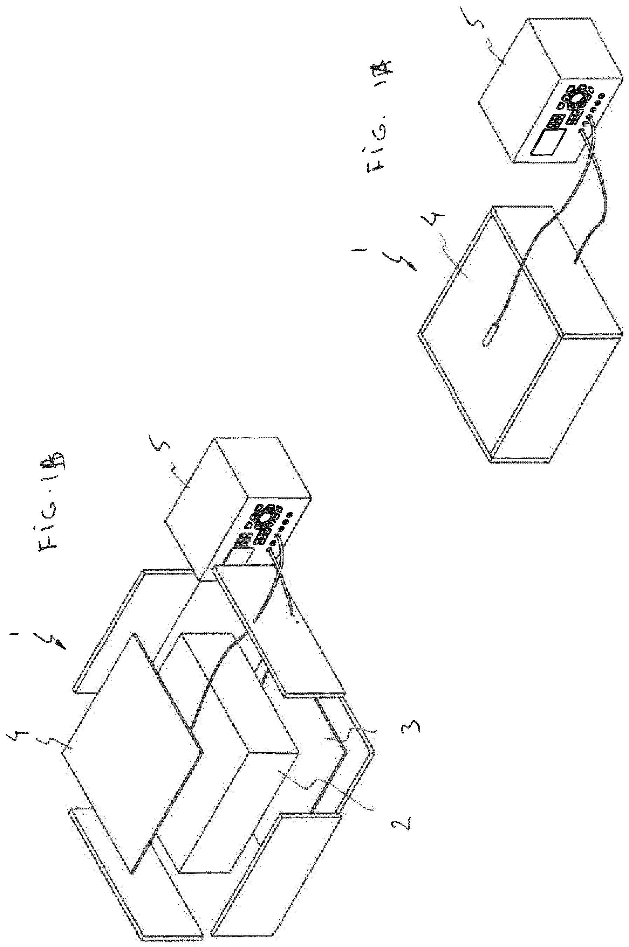

Figure 1A and 1B show a rectangular mould 1 for the production of a mycelium based insulation panel or building panel. A bed 2 of a particulate substrate material is placed within the mould 1, taking the shape of the panel to be produced. A first copper plate electrode 3 covers the full bottom of the mould 1. A second copper plate electrode 4 rests on top of the bed 2 of substrate material. The first and second plate electrodes 3, 4 are connected to an electric power generator 5. The power generator 5 generates a voltage over the thickness of the bed 2 of substrate material, so as to generate an electric current between the two electrodes 3, 4 crossing the complete bed 2.

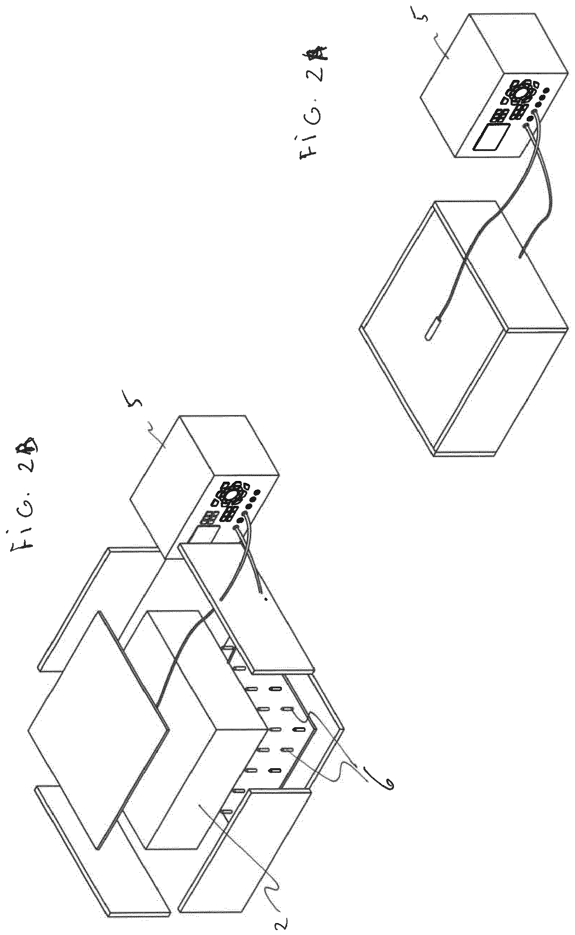

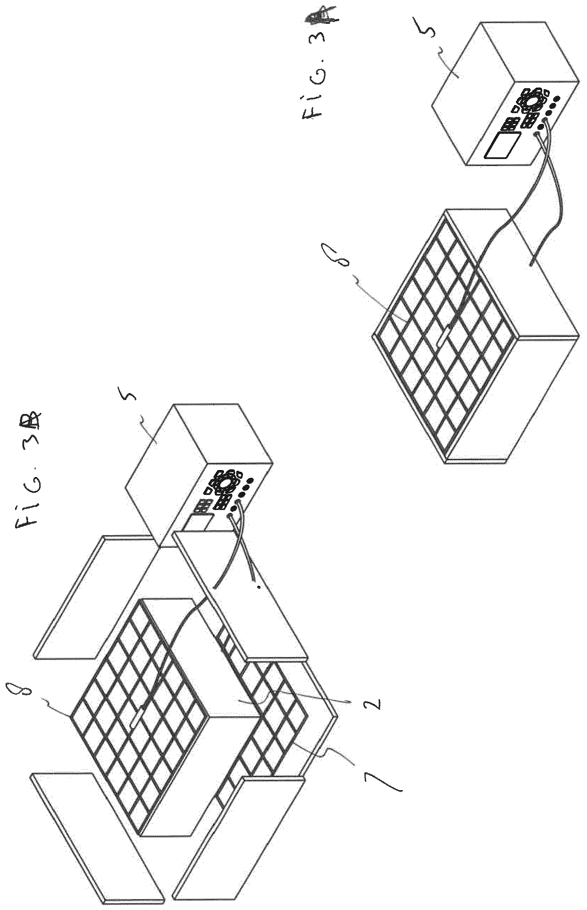

Figures 2A and 2B show an alternative embodiment with a number of pin electrodes 6 evenly distributed over the top and bottom surfaces of the bed 2 of substrate material. A third embodiment is shown in Figures 3A and 3B using grid electrodes 7, 8instead of plate electrodes.

### Example 1

A substrate material was prepared by mixing 50 wt% was sawdust with 50 wt% of mown biomass. The substrate material is sanitized in an autoclave and by adding an effective amount of H2O2, to inactivate pathogens and competing fungi strains. The substrate material was inoculated with an inoculum of the Ganoderma Lucidem strain and homogenized. Three samples were laid as a substrate bed in a mould as shown in Figure 1A. The moulds were 30 x 30 cm with a height of 12 cm. Two of the samples were laid on top of the lower electrode plate on the bottom of the mould. The top plate electrode was then put on top of the substrate bed.

The first sample was processed without being exposed to electric currents. The colonization step took about 96 h. The second sample was exposed to a voltage of 0,2 V and an electric current of 3 A. The colonization step of the second sample took about 46 h. The third sample was exposed to a voltage of 5 V and an electric current of 5 A. The colonization step took about 46 h. Hence, the duration of the colonization step was reduced with about 50 %.

### Example 2

After the colonization step, the substrate beds were exposed to stronger electric currents to deactivate the mycelium. Samples were exposed to a voltage of 200 V and a current of 50 A for at least 10 seconds. The blocks were then removed from the moulds in a room with a relative humidity of about 70% and a temperature of about 24°C. After four weeks there were still mycelial growth, which means that the mycelium was permanently deactivated. Samples in comparative tests without currents in a deactivating step, showed reactivated mycelial growth already after 6 hours.

The tests were repeated with other strains: Ganoderma lipsiense; Ganoderma adspersum; Pleurotus ostreatus; Pleurotus eryngii; Schizophyllum commune; Picnoporus sanguineus; and Stropharia rugosoannulata. The results were essentially the same.

## Claims

1. Process of making a mycelium based material comprising the steps of:
- depositing a substrate particulate material in a mould to form a substrate bed, the substrate particulate material comprising nutrients capable of being digested by a selected mycelium;
- inoculating the substrate particulate material with a mycelial inoculum to initiate colonization of the substrate bed by the mycelium;
- in a colonization step allowing the mycelium to colonize the inoculated substrate particulate material to form a mycelial network bonding the substrate particulate material;
- in a deactivating step deactivating the mycelium to inhibit further growth;
**characterized in that** electrical currents are used to control growth of the mycelium.

2. Process according to claim 1, wherein the electrical currents are generated across the substrate bed during the colonization step, the electrical currents having an amperage below 20 A, e.g., below 10 A.

3. Process according to claim 2, wherein a voltage is applied across the substrate bed, the voltage being below 1000 Volt, e.g., below 100 Volt.

4. Process according to any one of the preceding claims, wherein the electrical currents are generated across the substrate bed during the deactivating step, the electrical currents having an amperage of at least 20 A, e.g., at least 30 A.

5. Process according to claim 4, wherein a voltage is applied across the substrate bed, the voltage being at least 10 Volt, e.g., at least 100 Volt.

6. Process according to claim 4 or 5, wherein the electrical currents are generated for at least 5 seconds, e.g., at least 10 seconds.

7. Process according to any one of the preceding claims, wherein the electrical currents are generated in pulses or continuously.

8. Process according to any one of the preceding claims, wherein the electrical currents are generated between a first electrode at a bottom of the mould and a first electrode on top of the substrate bed.

9. Process according to claim 8, wherein the first and second electrodes are plate electrodes or grid electrodes covering the top and bottom surfaces of the substrate bed.

10. Process according to claim 8, wherein the electrodes are pin electrodes distributed over the top and bottom sides of the substrate bed and protruding into the substrate particulate material.

11. Process according to any one of the preceding claims, wherein the mycelium is selected from the following strains: Ganoderma Lucidem; Ganoderma lipsiense; Ganoderma adspersum; Pleurotus ostreatus; Pleurotus eryngii; Schizophyllum commune; Picnoporus sanguineus; Stropharia rugosoannulata.

12. Process according to any one of the preceding claims wherein during the colonization step conditions are controlled to promote mycelium growth without producing fruiting bodies.
